# EUROPÄISCHE PATENTSCHRIFT

(11) **EP 1 144 610 B1**
(45) Veröffentlichungstag und Bekanntmachung des Hinweises auf die Patenterteilung: **05.12.2007**
(21) Anmeldenummer: 00974311.3
(22) Anmeldetag: 05.09.2000
(51) Int. Cl.: C12N 15/82, A01H 5/00

(54) **VERFAHREN ZUR GESTEUERTEN NACH-ERNTE-PRODUKTION VON PROTEINEN IN PFLANZEN**
METHOD FOR CARRYING OUT THE CONTROLLED POST-HARVEST PRODUCTION OF PROTEINS IN PLANTS
PROCEDE DE PRODUCTION CONTROLEE POST-RECOLTE DE PROTEINES DANS DES PLANTES

(30) Priorität: 23.11.1999 DE 19956272
(43) Veröffentlichungstag der Anmeldung: 17.10.2001
(60) Teilanmeldung: 07002471.6
(73) Patentinhaber: MPB Cologne GmbH Molecular Plant & Protein Biotechnology, 51063 Köln (DE)
(72) Erfinder: DÜRING, Klaus, D-50226 Frechen-Königsdorf (DE); BÜLOW, Lorenz, D-38100 Braunschweig (DE)
(74) Vertreter: Schüssler, Andrea
(86) Internationale Anmeldenummer: PCT/DE2000/003119
(87) Internationale Veröffentlichungsnummer: WO 2001/038508

(56) Entgegenhaltungen:
- WO-A-95/00555
- WO-A-95/03690
- WO-A-97/22707
- WO-A-99/15668
- WO-A-99/23234
- GATZ C: "CHEMICAL CONTROL OF GENE EXPRESSION" ANNUAL REVIEW OF PLANT PHYSIOLOGY AND PLANT MOLECULAR BIOLOGY,ANNUAL REVIEWS INC,XX, Bd. 48, 1997, Seiten 89-108, XP000979191 ISSN: 1040-2519
- BUELOW, L., ET AL.: 'Induction of the maize GapC4 promoter in transgenic potato under anaerobiosis and in erwinia carotovora-inoculated tuber tissue' MOLECULAR PLANT-MICROBE INTERACTIONS Bd. 12, Nr. 3, 1999, Seiten 182 - 188, XP001006099
- MERCK CO.: 'Cat.No 1.13829.0001 - Experimental procedure and evaluation' MERCK CATALOGUE 2002, Seite 2 PG-S, XP003010408
- GERMAIN V. ET AL.: 'The role of sugars, hexokinase, and sucrose synthase in the determination of hypoxically induced tolerance to anoxia in tomato roots' PLANT PHYSIOL. Bd. 114, 1997, Seiten 167 - 175, XP003010409
- MERCK KGAA: 'Microbiology anaerobic incubation' MERCK KGAA, [Online] 2005, Seite 2 PG-S, XP003010410 Gefunden im Internet: <URL:http://www.merck.de/servlet/PB/menu/14 23000/index.html>
- FALLIK E. ET AL.: 'Tomato flavor and aroma quality as affected by a short anoxia treatment' 5TH POSTHARVEST SYMP. - ACTA HORT Bd. 682, 2005, Seiten 437 - 443, XP003010411
- CHANG W.W.P. ET AL.: 'Patterns of protein synthesis and tolerance of anoxia in root tips of maize seedlings acclimated to a low-oxygen environment, and identification of proteins by mass spectrometry' PLANT PHYSIOL. Bd. 122, Februar 2000, Seiten 295 - 317, XP003010412
- JACKSON M.B.: 'The impact of flooding stress on plants and crops' PLANTSTRESS 2006, Seiten 1 - 17, XP003010413
- MUJER C.V. ET AL.: 'Constitutive and inducible aerobic and anaerobic stress proteins in the echinochloa complex and rice' PLANT PHYSIOL. Bd. 101, 1993, Seiten 217 - 226, XP003010414
- ANDREWS D.L. ET AL.: 'The response of maize seedlings of different ages to hypoxic and anoxic stress' PLANT PHYSIOL. Bd. 105, 1994, Seiten 53 - 60, XP003010415
- RUSSEL D., SACHS M.: 'PROTEIN SYNTHESIS IN MAIZE DURING ANAEROBIC AND HEAT STRESS' PLANT PHYSIOL. Bd. 99, 1992, Seiten 415 - 420, XP002031336

## Beschreibung

Die vorliegende Erfindung betrifft ein Verfahren zur Gewinnung eines gewünschten Proteins aus einer transgenen Pflanze, wobei die Expression des für dieses Protein codierenden Gens erst nach der Ernte der Pflanze erfolgt und das Verfahren sich dadurch auszeichnet, daß die Expression des genannten Gens unter anaeroben Bedingungen erst nach der Ernte der Pflanze erfolgt.

In der Natur vorkommende Proteine sind oft nur in geringsten Mengen verfügbar, weisen aber hochinteressante Eigenschaften für Anwendungen als Wirk- und Werkstoffe auf. Da sie oft auch in rekombinanten Wirtssystemen, z.B. Bakterien, wie Escherichia coli, Bacillus subtilis, etc., nicht effizient zu wirtschaftlichen Bedingungen und in ausreichenden Mengen gewonnen werden können, ist in diesen Fällen eine kommerzielle Anwendung nicht realisierbar. Um immer komplexere und in niedrigen Organismen schwer oder gar nicht produzierbare Proteine herstellen zu können, sind außerdem zunehmend Zellen höherer Organismen mit ihrer inhärenten komplexen Proteinbiosynthese-Maschinerie als Wirtszellen erforderlich. Hier bieten sich seit einigen Jahren transgene Tiere, Pflanzen, Pilze, Moose, Algen, etc. als neue rekombinante Wirte an. Angesichts der Verfügbarkeit immer größerer Zahlen gut charakterisierter Proteine aus der molekularen Forschung wächst dieser Technologie eine immer größere Bedeutung für die Anwendung zu.

Die Expression von Fremd-Proteinen kann allerdings negative Auswirkungen auf die physiologische Konstitution des Wirtsorganismus haben und z.B. dessen Wachstum beeinträchtigen oder gar verhindern. Zudem ist es denkbar, daß z.B. bei dem Anbau von transgenen Pflanzen, die medizinisch aktive Proteine exprimieren, diese während des Wachstums der Pflanzen ein Gefährdungspotential für Organismen in der Umwelt darstellen. Daher hat die Nach-Ernte-Produktion von Fremd-Proteinen in transgenen Wirtsorganismen, insbesondere solchen, die landwirtschaftlich oder gartenbaulich kultiviert werden, eine große Bedeutung als technologischer Baustein auf dem Weg zu einer wirtschaftlichen und umweltverträglichen Gewinnung von Proteinen. Andere (bio-)chemische Substanzen sind ebenfalls in transgenen Wirtsorganismen durch die Expression von Enzymen, die in deren Biosyntheseweg involviert sind, darstellbar. Auch hier besteht allerdings das Risiko negativer Auswirkungen auf den Wirtsorganismus, z.B. Wachstumshemmungen, oder hinsichtlich der biologischen Sicherheit. Da die Produktion (bio-)chemischer Substanzen in Wirtsorganismen ebenfalls auf der Expression von Proteinen, insbesondere Enzymen, beruht, gelten hierfür die gleichen technologischen Vorgaben.

Bisher wurden Nach-Ernte-Produktionssysteme beschrieben, die auf Verwundung von Pflanzenmaterial, z.B. bei Tabakpflanzen, durch Zerkleinerung ihrer Blätter beruhen. Dadurch soll das gewünschte Protein während des Anbaus der Tabakpflanzen auf dem Feld noch nicht, sondern erst nach der Ernte vor dem Aufarbeitungsprozeß produziert werden. Dies ist aber nur schwer zu realisieren, da ein verwundungsinduzierbarer Promotor in transgenen Pflanzen im landwirtschaftlichen oder gartenbaulichen Anbau, z.B. in Tabak, schon bei Fraßschäden, Wind- oder Hagelschäden, Trittschäden, Maschineneinsatz, etc. induziert wird. Ein weiterer Nachteil des bisherigen Verfahrens stellt außerdem die schwer zu erreichende gleichmäßige Verteilung des Induktionsstimulus über die gesamte zu induzierende Pflanzenmasse dar.

Die WO 95/03690 offenbart Nach-Ernte-Verfahren bei denen die transgenen Pflanzen mit Konstrukten transformiert wurden, die das zu exprimierende Gen mit einem induzierbaren Promoter verknüpft enthalten. Ferner ist in der EP 0278658 eine anaerob regulierbare DNA-Sequenz beschrieben, die geeignet ist für die Expression von Genen in Pflanzen unter hypoxischen Bedingungen, d.h. unter Anwesenheit eines Restsauerstoffgehaltes von, vorzugsweise, 5% O₂.

Somit liegt der vorliegenden Erfindung das technische Problem zugrund, ein weiteres Nach-Ernte-Produktionsverfahren für ein gewünschtes Protein zur Verfügung zu stellen, dass gewährleistet, dass die Genexpression zuverlässig erst nach der Ernte erfolgt, ein Zerkleinern des Pflanzengewebes nicht.

Die Lösung dieses technischen Problems wird durch das in den Patentansprüchen angegebene erfindungsgemäße Verfahren erreicht.

In dem erfindungsgemäßen Verfahren erfolgt die Induktion der Expression eines Fremd-Gens in der Pflanze durch anaerobe Indukton.

Somit umfaßt die vorliegende Erfindung ein Verfahren zur Gewinnung eines gewünschten Proteins aus einer transgenen Pflanze, wobei die Expression des für dieses Protein codierenden Gens erst nach der Ernte der Pflanze erfolgt und das Verfahren sich dadurch auszeichnet, daß
(a) die transgene Pflanze das für das gewünschte Protein codierende Gen derart enthält, daß dessen Expression erst unter anaeroben Bedingungen erfolgt; und
(b) nach der Ernte der Pflanze durch Einleiten von Stickstoff oder Kohlendioxid die Expression anaerob induziert wird.

Verfahren zur Konstruktion der zur Durchführung des erfindungsgemäßen Verfahrens benötigten Nucleinsäurekonstrukte sind dem Fachmann bekannt und auch in gängigen Standardwerken beschrieben (vgl. z.B. Sambrook et al., 1989, Molecular Cloning, A Laboratory Manual, 2. Aufl. Cold Spring Harbor Laboratory Press, Cold Spring Harbor, NY). Das Nucleinsäurekonstrukt liegt vorzugsweise auf einem Vektor inseriert vor, wobei es sich bei dem Vektor vorzugsweise um ein Plasmid, ein Cosmid, ein Virus, einen Bacteriophagen oder einen anderen in der Gentechnik üblichen Vektor handelt. Diese Vektoren können weitere Funktionseinheiten besitzen, die eine Stabilisierung der Vektoren im Wirtsorganismus bewirken, wie einen bakteriellen Replikationsursprung oder die 2-Mikron-DNA zur Stabilisation in Saccharomyces cerevisiae. Ferner können "left border"- und "right border"-Sequenzen agrobakterieller T-DNA enthalten sein, wodurch eine stabile Integration in das Erbgut von Pflanzen ermöglicht wird. Ferner kann eine Terminationssequenz vorhanden sein, die der korrekten Beendigung der Transkription und der Addition einer Poly-A-Sequenz an das Transkript dient. Derartige Elemente sind in der Literatur beschrieben (vgl. Gielen et al., EMBO J. 8 (1989), 23-29) und sind beliebig austauschbar.

Zur Vorbereitung der Einführung eines Fremd-Gens in höhere Pflanzen stehen eine große Anzahl von Klonierungsvektoren zur Verfügung, die ein Replikationssignal für E.coli und ein Markergen zur Selektion transformierter Bakterienzellen enthalten. Beispiele für derartige Vektoren sind pBR322, pUC-Serien, M13mp-Serien, pACYC184, etc. Das Fremdgen kann an einer passenden Restriktionsschnittstelle in den Vektor eingeführt werden. Das erhaltene Plasmid wird für die Transformation von E.coli-Zellen verwendet. Transformierte E.coli-Zellen werden in einem geeigneten Medium gezüchtet, anschließend geerntet und lysiert, wodurch das Plasmid erhalten wird. Als Analysemethode zur Charakterisierung der gewonnenen Plasmid-DNA werden im allgemeinen Restriktionsanalysen, Gelelektrophoresen und weitere biochemisch-molekularbiologische Methoden eingesetzt. Nach jeder Manipulation kann die Plasmid-DNA gespalten und gewonnene DNA-Fragmente können mit anderen DNA-Sequenzen verknüpft werden. Jede Plasmid-DNA-Sequenz kann in den gleichen oder anderen Plasmiden kloniert werden.

Für die Einführung von DNA, z.B. in eine pflanzliche Wirtszelle, stehen eine Vielzahl von Techniken zur Verfügung. Diese Techniken umfassen die Transformation pflanzlicher Zellen mit T-DNA unter Verwendung von Agrobacterium tumefaciens oder Agrobacterium rhizogenes als Transformationsmittel, die Fusion von Protoplasten, die Injektion, die Elektroporation von DNA, die Einbringung von DNA mittels der biolistischen Methode sowie weitere Möglichkeiten.

Bei der Injektion und Elektroporation von DNA in Pflanzenzellen werden an sich keine speziellen Anforderungen an die verwendeten Plasmide gestellt. Es können einfache Plasmide, z.B. pUC-Derivate, verwendet werden. Sollen aber aus derartig transformierten Zellen ganze Pflanzen regeneriert werden, sollte ein selektierbarer Marker vorhanden sein. Je nach Einführungsmethode gewünschter Gene in die Pflanzenzelle können weitere DNA-Sequenzen erforderlich sein. Werden z.B. für die Transformation der Pflanzenzelle das Ti- oder Ri-Plasmid verwendet, so muß mindestens die rechte Begrenzung, häufig jedoch die rechte und linke Begrenzung der Ti- und Ri-Plasmid T-DNA als Flankenbereich mit den einzuführenden Genen verbunden werden.

Werden für die Transformation Agrobakterien verwendet, ist es günstig, die einzuführende DNA in spezielle Plasmide zu klonieren, insbesondere in einen intermediären oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf Agrobacterium tumefaciens übertragen werden. Binäre Vektoren können sowohl in E.coli als auch in Agrobakterien replizieren. Sie enthalten ein Selektionsmarker-Gen und einen Linker oder Polylinker, welche von der rechten und linken T-DNA Grenzregion eingerahmt werden.

Sie können direkt in die Agrobakterien transformiert werden. Das als Wirtszelle dienende Agrobakterium soll ein Plasmid, das eine vir-Region trägt, enthalten. Die vir-Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet.

Für den Transfer der DNA in die Pflanzenzelle können Pflanzen-Explantate zweckmäßigerweise mit Agrobacterium tumefaciens oder Agrobacterium rhizogenes kokultiviert werden. Aus dem infizierten Pflanzenmaterial, z.B. Blattstücke, Stengelsegmente, Wurzeln, Protoplasten oder Suspensions-kultivierte Pflanzenzellen können dann in einem geeigneten Medium, welches Antibiotika oder Biozide zur Selektion transformierter Zellen enthalten kann, wieder ganze Pflanzen regeneriert werden. Die so erhaltenen Pflanzen können dann auf Anwesenheit der eingeführten DNA untersucht werden. Andere Möglichkeiten der Einführung fremder DNA unter Verwendung des biolistischen Verfahrens oder durch Protoplasten-Fusion sind bekannt.

Alternative Systeme zur Transformation von monokotylen Pflanzen sind die Transformation mittels des biolistischen Ansatzes, die elektrisch oder chemisch induzierte DNA-Aufnahme in Protoplasten, die Elektroporation von partiell permeabilisierten Zellen, die Makroinjektion von DNA in Blütenstände, die Mikroinjektion von DNA in Mikrosporen und Pro-Embryonen, die DNA-Aufnahme durch keimende Pollen und die DNA-Aufnahme in Embryonen durch Quellung (zur Übersicht: Potrykus, Physiol. Plant (1990), 269 - 273). Während die Transformation dikotyler Pflanzen über Ti-Plasmid-Vektorsysteme mit Hilfe von Agrobacterium tumefaciens etabliert ist, weisen neuere Arbeiten darauf hin, daß auch monokotyle Pflanzen der Transformation mittels Agrobacterium basierender Vektoren zugänglich sind.

Bei den in dem erfindungsgemäßen Verfahren nützlichen transgenen Wirtsorganismen kann es sich prinzipiell um Pflanzen jeder beliebigen Pflanzenspezies handeln, d.h. sowohl monokotyle als auch dikotyle Pflanzen. Bevorzugt handelt es sich um Nutzpflanzen, insbesondere um Pflanzen, wie Weizen, Gerste, Reis, Mais, Zuckerrübe, Zuckerrohr, Kartoffel, Brassicacaeen, Leguminosen oder Tabak. Die für die Expression des gewünschten Proteins gewünschten Pflanzenteile betreffen prinzipiell jedes beliebige Pflanzenteil, jedenfalls Vermehrungsmaterial und Ernteprodukte dieser Pflanzen, z.B. Früchte, Samen, Knollen, Wurzelstöcke, Sämlinge, Stecklinge, etc.

Ferner kann in dem erfindungsgemäßen Verfahren jegliches Protein, insbesondere ein diagnostisches, ein therapeutisches und/oder ein Werkstoff-Protein hergestellt werden. Das Protein kann aus jeglichem Individuum, insbesondere dem Menschen oder einem Tier, stammen. Auch kann das Protein in Wildtyp- oder veränderter Form vorliegen. Ferner kann es ein Fusionsprotein oder ein Proteinfragment sein.

In einer bevorzugten Ausführungsform erfolgt bei dem erfindungsgemäßen Verfahren in Schritt (b) das Inkontaktbringen mit dem anaeroben Induktor über die den Wirtsorganismus umgebende Gasphase. Vorzugsweise erfolgt dies über eine Veränderung der den Wirtsorganismus umgebenden Gasphase, d.h. die Gasphase dient als Träger des Induktionsstimulus. Vorzugsweise führt die Veränderung der Gasphase im Wirtsorganismus zur Induktion, z.B. des betroffenenPromotors. Alleine aus physikalischen Gründen durch die Ausnutzung des Diffussionseffektes wird bereits nach einer bestimmten Zeit eine homogene Verteilung des Induktionsstimulus erreicht. Die Zeitspanne bis zur Einstellung dieser homogenen Verteilung wird durch den Einsatz einer aktiven Umwälzung der Gasphase im Reaktionsraum wesentlich verkürzt. Somit wird insbesondere bei dicht aneinanderliegenden Zellmassen, wie bei Pflanzenblättern, Algen- oder Moosgewebe, eine gleichmäßige und schnelle Durchdringung erreicht. Insbesondere bei kompakten Geweben, z.B. Kartoffelknollen, ist die erfindungsgemäße schnelle Durchdringung des Gewebes von großem Vorteil.

Geeignete gasförmige Induktoren sowie Bedingungen zum möglichst effizienten Austausch der Gasphase sind dem Fachmann bekannt. Es wird auf das Anaerocult-System (Merck, Darmstadt, Deutschland) verwiesen, das ein anaerobes Milieu, in dem Sauerstoff gebunden und CO₂ freigesetzt werden, erzeugt. In diesem System wird der GapC4 Promoter aus Mais anaerob durch die CO₂-Atmosphäre induziert (Bülow, L. et al., Molecular Plant-Microbe Interactions (1999), 182-188). Ebenso wird der gleiche Effekt durch Einleitung von technischem Stickstoff erreicht.

In einer besonders bevorzugten Ausführungsform des erfindungsgemäßen Verfahrens ist das für das gewünschte Protein codierende Gen mit einem induzierbaren Promotor verknüpft. Geeignete Promotoren und die hierfür in Frage kommenden Inhibitoren (gasförmige, flüssige oder feste, flüchtige Verbindungen) sind dem Fachmann bekannt. Hierzu zählen z.B. die vorstehend beschriebenen Systeme GapC4 Promotor / CO2 (Bülow et al., supra), der Adh1 Promotor aus Mais / Anaerobiose (Ellis et al., EMBO J. 6 (1987), 11-16).

Vorzugsweise handelt es sich bei dem Promotor um einen unter aeroben Bedingungen inaktiven Promotor, wie den GapC4-Promotor oder den Adhl-Promotor und die Induktion erfolgt über eine Veränderung der Gasphase derart, daß es sich um einen Sauerstoffentzug handelt. Besonders bevorzugt ist hierbei der anaerob induzierbare GapC4 Promotor, (DE 195 47 272), z.B. in Verbindung mit abgeerntetem transgenen Pflanzengewebe, z.B. transgenen Kartoffelknollen. Die meisten pflanzlichen Promotoren werden unter anaeroben Bedingungen abgeschaltet, während der GapC4 Promotor unter aeroben Bedingungen ausgeschaltet ist und durch einfachen Sauerstoffentzug angeschaltet wird (Bülow et al., supra). Dies wird z.B. durch die Einleitung von Stickstoff oder Kohlendioxid in den Reaktions- oder Lagerraum erreicht. Innerhalb weniger Stunden wird somit auch in einer intakten Kartoffelknolle ein vollständig anaerobes Milieu erreicht, wodurch die Promotorinduktion und Fremdproteinexpression erfolgt.

In einer weiteren bevorzugten Ausführungsform des erfindungsgemäßen Verfahrens erfolgt die Induktion der Expression des für das gewünschte Protein codierenden Gens durch Aufhebung der funktionellen Hemmung der Transkription und/oder Translation. Ein Beispiel für eine transkriptionale Hemmung ist das HRT-Protein (Repressor) aus Gerste, welches an das "Gibberellinphytohormone response element" des α-Amylase-Promotors aus Gerste bindet (Raventos, D. et al., J. Biol. Chem. 273 (1998), 23313-23320). Wird dieser Repressor unter der Kontrolle des CaMV 35S Promotors unter aeroben Bedingungen in einer transgenen Pflanze exprimiert, so bindet er an die Targetsequenz, welche zwischen dem für das gewünschte Protein codierenden Gen und seinem Promotor eingefügt ist. Bei Erzeugung anaerober Bedingungen wird der 35S-Promotor abgeschaltet und die Repressorneusynthese unterbunden, folglich das vorstehende Gen durch den Abbau des vorhandenen Repressors aktiviert.

Ein Beispiel für die translationale Hemmung ist die Repression der Translation des Ferritin-Transkripts in Weizenkeimextrakten durch die Expression eines 90 kDa Repressors aus Rattenleber, welcher an die 5'-untranslatierte Region der Ferritin-mRNA bindet (Brown, P.H., J. Biol. Chem. 264 (1989), 13383-13386). Der Repressor der Ferritin-mRNA wird vor die für das gewünschte Protein codierende cDNA-Sequenz inseriert und der Repressor wird in der transgenen Pflanze exprimiert.

Auch kann die Aktivität des Repressors durch einen Induktor oder Inhibitor direkt kontrolliert werden.

In einer besonders bevorzugten Ausführungsform ist das erfindungsgemäße Verfahren dadurch gekennzeichnet, daß das für das gewünschte Protein codierende Gen derart mit einem Promotor funktionell verknüpft ist, daß zwischem dem Promotor und dem Gen eine Nucleinsäure inseriert ist, die dadurch gekennzeichnet ist, daß (a) sie die Transkription und/oder Translation des Gens verhindert; und (b) mittels Induktion exzisiert werden kann, was zur Expression des Gens führt. Somit wird im nicht-induzierten Zustand die Transkription oder Translation des gewünschten Gens unterbunden und damit auch das von diesem Gen codierte Fremd-Protein nicht gebildet. Bei einer noch mehr bevorzugten Ausführungsform des erfindungsgemäßen Verfahrens ist die inserierte Nukleinsäure durch eine reprimierte und induzierbare Rekombinase exzisierbar. Das für die Rekombinase codierende Gen kann dabei getrennt in die Wirtspflanze eingebracht werden oder selbst auf der inserierten Nucleinsäure lokalisiert sein. Erst nach Aktivierung der Rekombinase durch einen (bio-)chemischen, physikalischen oder genetischen Induktor erfolgt eine ortsspezifische Rekombination und damit eine Exzision dieser Nukleinsäuresequenz, wodurch das gewünschte Gen direkt stromabwärts zum Promotor lokalisiert wird, was die Transkription und Translation und somit die Fremd-Protein-Biosynthese initiiert. Hinsichtlich der die Rekombinase aktivierenden Induktoren wird auf vorstehende Ausführungen bezüglich Promotor Induktionssysteme verwiesen. Besonders wird erwähnt, daß das Rekombinase-Gen auf einem Virus, z.B. TMV, liegen kann und erst nach Infektion des Wirtsorganismus aktiviert wird.

Eine weitere alternative bevorzugte Ausführungsform des erfindungsgemäßen Verfahrens ist dadurch gekennzeichnet, daß die Expression des für das gewünschte Protein codierenden Gens durch die Aufhebung der Wirkung eines transkriptionalen, posttranskriptionalen, translationalen oder posttranslationalen Repressors erfolgt, wobei die Ablösung des Repressors von der gebundenen Nukleinsäure- bzw. Proteinsequenz durch einen externen Stimulus induziert wird. Es wird auf vorstehende Ausführungen bezüglich der Aufhebung der funktionellen Hemmung der Transkription und/oder Translation verwiesen.

Schließlich betrifft die vorliegende Erfindung ein Verfahren, bei dem verschiedene der vorstehend beschriebenen Induktionsmechanismen, z.B. als Zweikomponenten-System, kombiniert werden. Dadurch kann eine noch restriktivere Regulierung der Fremd-Genexpression, z.B. in transgenen Pflanzen, und damit ein noch weiter gesteigertes Maß der biologischen Sicherheit erreicht werden, gleichzeitig können aber auch potentielle negative Effekte auf die Physiologie der Pflanze unterbunden werden. Beispielsweise kann dazu ein durch eine gasförmige Substanz induzierbarer Promotor, z.B. der anaerob induzierbare GapC4-Promotor aus Mais, oder ein auf einem Repressor basierendes System mit einem anderen System, z.B. einem auf Rekombination basierenden System, kombiniert werden, sodaß die Transkription des betreffenden Fremd-Gens ausgehend von dem induzierbaren Promotor erst nach erfolgter Zusammenführung von Promotor und Gen durch gesteuerte Rekombination ermöglicht wird. Als Rekombinationssystem kann z.B. das Rekombinase-LBD-System (WO 95/00555) verwendet werden, das aus einer 5' Rekombinationsstelle für die Rekombinase, der für das Rekombinase-LBD-Protein codierenden cDNA und einer 3' Rekombinationsstelle für die Rekombinase besteht. Bei diesem Zweikomponenten-System ist der Promotor unter aeroben Bedingungen inaktiv. Die Rekombinase ist durch die Fusion mit der LBD-Domäne ebenfalls inaktiviert. Somit herrscht doppelte Repression vor. Unter landwirtschaftlichen und gartenbaulichen Bedingungen erfolgt somit keine Expression des Fremd-Gens, selbst wenn eines der beiden Repressionssysteme durch einen Umweltstimulus nicht vollständig blockiert sein sollte. Dieser negative Effekt würde dann durch das zweite (Repressions)system aufgefangen. Nach der Ernte des Pflanzenmaterials wird zur Fremdprotein-Produktion des an die LBD-Domäne bindende Induktor zugegeben und außerdem werden anaerobe Bedingungen in der Reaktionskammer hergestellt. Dadurch wird einerseits die Rekombinase aktiviert, folglich an den beiden Rekombinationsstellen das die Rekombinase codierende cDNA-Fragment herausgeschnitten und somit das zu exprimierende Gen direkt an den anaeroben Promotor lokalisiert. Durch Erzeugung anaerober Bedingungen wird nunmehr die Transkription und Translation des zu exprimierenden Gens ermöglicht und das Fremd-Protein unter kontrollierten Bedingungen produziert.

Die folgenden Beispiele erläutern die Erfindung.

### Beispiel 1: Anaerobe Nach-Ernte-Produktion von scFv in transgenen Kartoffeln

Der anaerob induzierbare GapC4 Promotor (DE 195 47 272) wurde mittels einer PCR-Reaktion derart modifiziert, daß er am 5'-Ende eine HincII-Restriktionsschnittstelle und eine Ncol-Restriktionschnittstelle erhielt. Für die PCR-Reaktion wurde folgendes

Primerpaar verwendet:

| | |
|---|---|
| HincII-pGapC4 Primer: | CATGTCAACACATAAGGAAGAAGAGGTAGAAAG |
| pGapC4-NcoI Primer: | CATGCCATGGATCGATGACGGGGTTGGCGAGTGTG |

Die von Artsaenko et al., Molecular Breeding 4 (1998), 313-319 beschriebene cDNA, die für einen im endoplasmatischen Retikulum lokalisierten scFv-Antikörper codiert, wurde mittels einer Linker-Ligation (am 5'-Ende mit CATGCCATGGCATG, [5'phosphoryliertes Oligonukleotid]; am 3'-Ende mit GCTCTAGAGC [5' phosphoryliertes Oligonukleotid] derart modifiziert, daß sie am 5'-Ende eine NcoI-Restriktionsschnittstelle und am 3'-Ende eine XbaI-Restriktionsschnittstelle aufwies. Aus dem Plasmid pRT100 (Töpfer et al., Nucleic Acids Research 15 (1987), 5890) wurde der CaMV 35S Promotor mittels Restriktionsverdau mit HincII und XbaI entfernt. Stattdessen wurden die beiden vorstehend beschriebenen Nucleinsäurefragmente, die für den GapC4 Promotor und den scFv-Antikörper codieren, inseriert. Nach partieller Spaltung mit HindIII wurde die Expressionskassette isoliert und in den binären Vektor pSR 8-30 inseriert (Düring et al., Plant Journal 3 (1993), 587-598; Porsch et al., Plant Molecular Biology 37 (1998), 581-585). Es wurde der Expressionsvektor pSR 8-30/Gap-scFv(ox) erhalten.

Dieser wurde zur Transformation von E.coli SM10 (Koncz und Schell, Molecular and General Genetics 204 (1986), 383-296) verwendet. Transformanten wurden mit Agrobacterium GV 3101 (Koncz und Schell, supra) gemischt und über Nacht bei 28°C inkubiert (Koncz et al., Proc.Natl.Acad.Sci.USA 84 (1987), 131-135). Es wurde auf Carbenicillin selektioniert, wobei das hierfür notwendige bla-Gen in den vorstehenden Expressionsvektoren vorliegt. Selektionsklone von Agrobacterium tumefaciens wurden auf abgeschnittenen und mehrfach an der Mittelrippe eingeritzten Blättern der Kartoffelpflanze cv. Désirée aufgebracht und die Blätter wurden 2 Tage bei 20°C im Dunkeln inkubiert. Danach wurden die Agrobakterien abgewaschen und den Kartoffelblättern Pflanzenwuchsstoffe zugesetzt, so daß bevorzugt Sprosse regenerierten. Ferner wurden durch die Zugabe von Kanamycin (100 mg/ml) in das Pflanzenmedium nicht-transformierte Zellen in den Kartoffelblättern abgetötet. Heranwachsende Sprosse wurden abgeschnitten und in dem Medium ohne Pflanzenwachstumsstoffe, aber mit Kanamycin, bewurzelt. Die weitere Kultivierung der Kartoffelpflanzen erfolgte in üblicher Weise.

Zum Nachweis der Expression des scFv-Antikörpers wurde abgeschnittenes Blattmaterial oder intaktes oder geschnittenes Knollenmaterial mittels des "Anaerocult"-Systems (Fa. Merck, Darmstadt, Deutschland) wie bei Bülow et al. (1999), Supra beschrieben, induziert. Nach 40 Stunden wurde das Blattmaterial entnommen und gemörsert. Der Nachweis des exprimierten scFv-Antikörpers wurde über den enthaltenen c-myc Tag mittels des monoklonalen Antikörpers 9E10-IgG (Cambridge Research Chemicals, Northwich, Cheshire, UK) oder Protein L (Clontech, Palo Alto, CA., USA) im Western Blot bzw. ELISA erbracht. Hierzu wurde das Gesamtprotein des Kartoffelmaterials isoliert und in die entsprechenden Nachweisverfahren eingesetzt.

Als expressionspositiv ermittelte transgene Kartoffelpflanzen wurden im Gewächshaus (im Topf oder im Erdbeet) oder im Freiland unter üblichen gartenbaulichen bzw. landwirtschaftlichen Bedingungen angebaut. Die Knollen wurden nach üblicher Handhabung geerntet und gelagert. Für die Nach-Ernte-Produktion des scFv-Antikörpers wurden die Knollen in einen Reaktionsbehälter aus Stahl (oder Kunststoff) verbracht, der unten ein Gaszufuhrventil und oben ein Gasabführventil aufwies. Die Raumluft im Behälter wurde schnell von technischem Stickstoff (oder Kohlendioxid) verdrängt. Unter langsamem Gasstrom ( 1 m³ Gaszufuhr pro Stunde pro m² Grundfläche) wurde eine konstante Zusammensetzung der Gasphase im Reaktionsbehälter eingestellt. Nach 40 Stunden wurden die Knollen aus dem Reaktionsbehälter entnommen, homogenisiert, der Festanteil abzentrifugiert und der wäßrige Überstand der chromatographischen Aufreinigung des scFv-Antikörpers zugeführt. Durch Vergleich von Proben vor und nach Sauerstoffentzug konnte gezeigt werden, daß vor der Verdrängung des Sauerstoffs kein scFv-Antikörper, danach jedoch signifikante Mengen davon produziert wurden.

### Beispiel 2: Rekombinations-vermittelte Nach-Ernte-Produktion in transgenen Kartoffeln

Die in Artsaenko et al. (1998) beschriebene cDNA, die für einen im endoplasmatischen Retikulum lokalisierten scFv-Antikörper codiert, wurde mittels einer Linker-Ligation wie in Beispiel 1 beschrieben, derart modifiziert, daß sie am 5' Ende eine NcoI-Restriktionsschnittstelle und am 3' Ende eine XbaI-Restriktionsschnittstelle aufwies. Diese Nucleinsäuresequenz wurde in das Plasmid pRT 100 in die Polylinkersequenz inseriert. Es wurde das Plasmid pRT100/scFv(ox) erhalten. In die NcoI-Restriktionsstelle von pRT100/scFv(ox) wurde eine synthetische Nucleinsäure inseriert, die zwei FRT-Rekombinationsstellen (Buchholz et al., Nucleic Acids Res. 24 (1996), 3118-3119) enthält. Es wurde das Plasmid pRT 100/FRT-scFv(ox) erhalten. Die codierende cDNA für das FLP-Rekombinase-LBD-Fusionsprotein (WO 95/00555) wurde als PCR-adaptiertes Ncol-XbaI-Fragment in die NcoI-Schnittstelle von pRT 100 in der Sense-Orientierung hinter den CaMV 35S Promotor (Odell et al., Nature 313 (1985), 810-812) inseriert (Plasmid pRT 100/FLP). Folgendes Primer-Paar wurde hierfür verwendet:

| | |
|---|---|
| NcoI-PLP-LBD Primer: | CATGCCATGCCACAATTTGATATATTATGTAAAAC |
| FLP-LBD-XbaI Primer: | GCTCTAGATCAGACTGTGGCAGGGAAACCCTC |

Zwischen die beiden FRT-Rekombinationsstellen in pRT 100/FRT-scFv(ox) wurde die Expressionskassette aus pRT 100/FLP als partiell verdautes PstI-Fragment inseriert. Dadurch wurde das Plasmid pRT 100/rec-scFv(ox) erhalten.

Nach Spaltung mit HindIII wurde die Expressionskassette aus pRT 100/rec-scFv(ox) isoliert und in den binären Vektor pSR 8-30 inseriert (Düring et al., Plant Journal 3 (1993), 587-598; Porsch et al., Plant Molecular Biology 37 (1998), 581-585). Es wurde der Expressionsvektor pSR 8-30/rec-scFv(ox) erhalten. Die Erzeugung transgener Kartoffelpflanzen erfolgte wie im vorstehenden Beispiel 1 beschrieben.

Zum Nachweis der Expression des scFv-Antikörpers wurden in vitro kultivierte Kartoffelpflanzen auf Medium mit 10⁻⁶ M Östradiol (Sigma Chemicals, St. Louis, MO, USA) umgesetzt und für 2 Tage kultiviert. Danach wurde das Pflanzenmaterial geerntet und gemörsert. Der Nachweis exprimierter scFv-Antikörper wurde wiederum über den enthaltenen c-myc Tag (vgl. supra) oder Protein L im Western Blot bzw. ELISA durchgeführt. Hierzu wurde das Gesamtprotein des Kartoffelmaterials isoliert und in die entsprechenden Nachweisverfahren eingesetzt.

Kartoffelpflanzen von als expressionspositiv ermittelten Linien wurden aus der Klonalen in vitro-Kultur genommen und im Gewächshaus ausgepflanzt. Diese Einzelpflanzen waren noch nicht mit Östradiol behandelt, so daß noch keine Rekombination stattgefunden hatte. Gleichermaßen erfolgte der Anbau im Feld nach üblichen landwirtschaftlichen Bedingungen. Die Knollen wurden nach üblicher Handhabung geerntet und gelagert. Für die Nach-Ernte-Produktion des scFv-Antikörpers wurden die Knollen mit einer vernebelten Lösung von 10⁻⁶ M Östradiol in einem Reaktionsbehälter inkubiert. Die technische Ausführung erfolgte, wie im vorstehenden Beispiel 1 beschrieben. Nach 40 Stunden wurden die Knollen aus dem Reaktionsbehälter entnommen, homogenisiert, der Festanteil wurde abzentrifugiert und der wäßrige Überstand der chromatographischen Aufreinigung des scFv-Antikörpers zugeführt. Durch Vergleich von Proben vor und nach Kontakt mit Östradiol konnte gezeigt werden, daß vor dem Inkontaktbringen mit Östradiol kein scFv-Antikörper, danach jedoch signifikante Mengen davon produziert wurden.

## Patentansprüche

1. Verfahren zur Gewinnung eines gewünschten Fremdproteins aus einer transgenen Pflanze, wobei die Expression des für dieses Fremdprotein codierenden Gens erst nach der Ernte der Pflanze erfolgt, **dadurch gekennzeichnet, daß**
(a) die transgene Pflanze das für das gewünschte Fremdprotein codierende Gen derart enthält, daß dessen Expression erst durch eine anaerobe Induktion erfolgt; und
(b) die Induktion der Expression unter anaeroben Bedingungen nach der Ernte der Pflanze oder Teilen davon in einem Reaktionsbehälter durch den Austausch der die Pflanze umgebende Gasphase erfolgt, wobei der Reaktionsbehälter ein Gaszufuhr- und ein Gasabfuhrventil aufweist, die Raumluft im Reaktionsbehälter durch Einleiten von Stickstoff oder Kohlendioxid verdrängt wird und die Fremdproteinexpression in einem vollständig anaeroben Milieu erfolgt.

2. Verfahren nach Anspruch 1, wobei das für das gewünschte Fremdprotein codierende Gen mit einem induzierbaren Promotor funktionell verknüpft ist.

3. Verfahren nach Anspruch 2, wobei der Promotor ein unter aeroben Bedingungen inaktiver Promotor ist.

4. Verfahren nach Anspruch 3, wobei der Promotor der GapC4 Promotor ist.

5. Verfahren nach Anspruch 1, wobei die Induktion der Expression des für das gewünschte Fremdprotein codierenden Gens durch Aufhebung der funktionellen Hemmung der Transkription und/oder Translation erfolgt.

6. Verfahren nach Anspruch 5, wobei das für das gewünschte Fremdprotein codierende Gen so mit einem Promotor funktionell verknüpft ist, daß zwischen dem Promotor und dem Gen eine Nucleinsäure inseriert ist, die **dadurch gekennzeichnet ist, daß**
(a) sie die Transkription und/oder Translation des Gens verhindert; und
(b) sie nach Induktion exzisiert werden kann, was zur Expression des Gens führt.

7. Verfahren nach Anspruch 6, wobei die Nucleinsäure eine durch eine induzierbare Rekombinase exzisierbare Nucleinsäure ist.

8. Verfahren nach Anspruch 1, wobei die Expression des für das gewünschte Fremdprotein codierenden Gens durch die Aufhebung der Wirkung eines transkriptionalen, posttranskriptionalen, translationalen oder posttranslationalen Repressors erfolgt.

9. Verfahren nach einem der Ansprüche 1 - 8, wobei das Verfahren eine Kombination aus mindestens zwei der in den Ansprüchen 2, 5, 6 und 8 definierten Verfahren ist.

10. Verfahren nach einem der Ansprüche 1 - 9, wobei die transgene Pflanze eine Nutzpflanze ist.

11. Verfahren nach Anspruch 10, wobei die Nutzpflanze Weizen, Gerste, Mais, Zuckerrübe, Zuckerrohr, Kartoffel, eine Brassicacaee, eine Leguminose oder Tabak ist.

## Claims

1. A method of obtaining a desired foreign protein from a transgenic plant, the gene coding for this foreign protein being only expressed after the harvest of the plant, **characterized in that**
(a) the transgenic plant contains the gene coding for the desired foreign protein such that the expression thereof only takes place by an anaerobic induction; and
(b) the expression is only induced under anaerobic conditions after the harvest of the plant or parts thereof in a reaction vessel by the exchange of the gas phase surrounding the plant, the reaction vessel having a gas supply valve and a gas discharge valve, the ambient air in the reaction vessel being displaced by the introduction of nitrogen or carbon dioxide, and the foreign protein expression taking place in a fully anaerobic environment.

2. The method according to claim 1, wherein the gene coding for the desired foreign protein is functionally linked with an inducible promoter.

3. The method according to claim 2, wherein the promoter is a promoter inactive under aerobic conditions.

4. The method according to claim 3, wherein the promoter is the GapC4 promoter.

5. The method according to claim 1, wherein the expression of the gene coding for the desired foreign protein is induced by eliminating the functional inhibition of transcription and/or translation.

6. The method according to claim 5, wherein the gene coding for the desired foreign protein is functionally linked with a promoter so as to insert a nucleic acid between promoter and gene, which is **characterized in that**
(a) it prevents the transcription and/or translation of the gene; and
(b) it can be excised following induction, which results in the expression of the gene.

7. The method according to claim 6, wherein the nucleic acid is a nucleic acid excisable by an inducible recombinase.

8. The method according to claim 1, wherein the gene coding for the desired foreign protein is expressed by eliminating the effect of a transcriptional, posttranscriptional, translational or posttranslational repressor.

9. The method according to any of claims 1 to 8, wherein the method is a combination of at least two of the methods defined in claims 2, 5, 6 and 8.

10. The method according to any of claims 1 to 9, wherein the transgenic plant is a useful plant.

11. The method according to claim 10, wherein the useful plant is wheat, barley, corn, sugar beet, sugar cane, potato, a brassicaceae, a legume or tobacco.

## Revendications

1. Procédé de production de protéines étrangères souhaitées à partir d'une plante transgénique, dans laquelle l'expression du gène codant pour la protéine étrangère ne se produit qu'après la récolte de la plante, **caractérisé en ce que** :
a) la plante transgénique renferme le gène codant pour la protéine étrangère souhaitée de telle manière que son expression résulte d'abord par une induction anaérobie ; et
b) l'induction de l'expression dans des conditions anaérobie résulte après la récolte de la plante ou bien des parties de celle-ci par l'échange de la phase gazeuse entourant la plante dans un récipient de réaction, le récipient de réaction comprenant une valve d'entrée de gaz et une valve de sortie de gaz, la partie gazeuse dans le récipient de réaction étant alimentée en azote ou en gaz carbonique par des conduits et l'expression de la protéine étrangère résulte dans un milieu complètement anaérobie.

2. Procédé selon la revendication 1, **caractérisé en ce que** le gène codant pour la protéine étrangère souhaitée est lié fonctionnellement à l'aide d'un promoteur inductible.

3. Procédé selon la revendication 2, **caractérisé en ce que** le promoteur est un promoteur inactif dans des conditions anaérobies.

4. Procédé selon la revendication 3, **caractérisé en ce que** le promoteur est le promoteur GapC4.

5. Procédé selon la revendication 1, **caractérisé en ce que** l'induction de l'expression du gène codant pour la protéine étrangère souhaitée résulte de la suppression du ralentissement fonctionnel de la transcription et/ou de la translation.

6. Procédé selon la revendication 5, dans lequel le gène codant pour la protéine étrangère souhaitée est fonctionnellement lié avec un promoteur de telle façon qu'un acide nucléique est inséré entre le promoteur et le gène, qui **caractérisé en ce que** :
a) il prévient la transcription et/ou la translation du gène,
b) il peut être excisé après induction, ce qui conduit à l'expression du gène.

7. Procédé selon la revendication 6, **caractérisé en ce que** l'acide nucléique est un acide nucléique détachable par une recombinase inductible.

8. Procédé selon la revendication 1, **caractérisé en ce que** l'expression du gène codant pour la protéine étrangère souhaitée résulte de la suppression de l'action d'un répresseur de transcription, de post-transcription, de translation ou de post-translation.

9. Procédé selon l'une des revendications 1 à 8, **caractérisé en ce que** le procédé est une combinaison d'au moins deux des procédés définis dans les revendications 2, 5, 6 et 8.

10. Procédé selon l'une des revendications 1 à 9, **caractérisé en ce que** la plante transgénique est une plante utilitaire.

11. Procédé selon la revendication 10, **caractérisé en ce que** la plante utilitaire est le blé, l'orge, le maïs, la betterave, la canne à sucre, la carotte, un crucifère, une légumineuse ou le tabac.
